# EUROPEAN PATENT APPLICATION

(11) **EP 2 590 096 A2**
(43) Date of publication of application: **08.05.2013**
(21) Application number: 12171623.7
(22) Date of filing: 12.06.2012
(51) Int. Cl.: G06F 19/00

(54) **Method and apparatus for displaying analysis result of medical measured data**

(30) Priority: 02.11.2011 KR 20110113532
(71) Applicant: Samsung Electronics Co., Ltd., Suwon-si, Gyeonggi-do, 443-742 (KR)
(72) Inventor: Kwon, Keun-Joo, Seoul (KR); Woo, Kyoung-Gu, Seoul (KR); Kim, Ye-Hoon, Seoul (KR); Hong, Seok-Jin, Gyeonggi-do (KR)
(74) Representative: Grünecker, Kinkeldey, Stockmair & Schwanhäusser

(57) **Abstract**

A method and apparatus to analyze received measured medical data and display correlated analysis results are provided. A method of displaying an analysis result of measured data, includes analyzing measured data received for disease diagnosis and deriving results of analysis items based on the analyzed measured data. The method further includes displaying the results by setting each of the analysis items as a variant and allowing correlated ones of the analysis items to be disposed adjacent to each other.

## Description

### CROSS-REFERENCE TO RELATED APPLICATION

This application claims the benefit under 35 U.S.C. §119(a) of a Korean Patent Application No. 10-2011-0113532, filed on November 2, 2011, in the Korean Intellectual Property Office, the entire disclosure of which is incorporated herein by reference for all purposes.

### BACKGROUND

### 1. Field

The following description relates to a method and apparatus for a user interface in a diagnostic medical device.

### 2. Description of the Related Art

In modern medical science, diseases are diagnosed with the help of various kinds of medical devices. These medical devices include, for example, an electrocardiogram (ECG) measuring device, a sphygmomanometer, a thermometer, an oxygen saturation measuring device, a magnetic resonance imaging (MRI) device and a computed tomography (CT) device, which directly measure patients' biological signals. In other examples, the medical devices include a blood glucose measuring device, a blood test meter and a diabetic test meter, which obtain results by collecting substances from patients' bodies and performing biochemical tests on the collected body substances. Medical staffs diagnose patients' diseases by observing or analyzing measured data using the medical devices, and accordingly, make appropriate prescriptions for patients.

As measured data are accumulated, it becomes difficult to read a result of the measured data at a glance. This is the case when a method of displaying the result includes, for example, listing only measured data. Hence, it is necessary to provide a method of effectively displaying the result.

### SUMMARY

The following description relates to a method and apparatus to display an analysis result of medical measurement data, which display the analysis result of analysis items so that correlated analysis items are disposed adjacent to each other.

In one general aspect, there is provided a method of displaying an analysis result of measured data including analyzing measured data received for disease diagnosis and deriving results of analysis items based on the analyzed measured data. The method further includes displaying the results by setting each of the analysis items as a variant and allowing correlated ones of the analysis items to be disposed adjacent to each other.

The displaying of the results includes displaying the results in a radial graph in which variants include axes, and the analysis items are disposed based on correlations between the analysis items, corresponding to the respective axes.

The analysis items include kinds of possible diseases, and are disposed based on correlations between the diseases.

The analysis items include pathological causes of possible diseases, and are disposed based on correlations between the pathological causes of the diseases.

The analysis items include positions of possible diseases, and are disposed based on physical proximities at the positions of the diseases.

The displaying of the results includes displaying the results accumulated as time elapses.

The deriving of the results includes subdividing the analyzed measured data into results of first analysis items and second analysis items.

The displaying of the results includes three-dimensionally displaying the results by respectively setting the first and second analysis items to X and Y axes, and the first or second analysis items are disposed adjacent to each other based on their correlations between each other.

The results are in forms of at least one of accuracy, confidence, probability and truth value.

In another general aspect, an apparatus configured to display an analysis result of measured data, includes an analysis unit configured to analyze measured data received for disease diagnosis, and derive results of analysis items based on the analyzed measured data. The apparatus further includes a control unit configured to display the results by setting each of the analysis items as a variant and allowing correlated ones of the analysis items to be disposed adjacent to each other.

The control unit displays the results in a radial graph in which variants include axes, and the analysis items are disposed based on correlations between the analysis items, corresponding to the respective axes.

The analysis items include kinds of possible diseases, and are disposed based on correlations between the diseases.

The analysis items include pathological causes of possible diseases, and are disposed based on correlations between the pathological causes of the diseases.

The analysis items include positions of possible diseases, and are disposed based on physical proximities at the positions of the diseases.

The control unit displays the results accumulated as time elapses.

The control unit subdivides the analyzed measured data into results of first analysis items and second analysis items.

The control unit three-dimensionally displays the results by respectively setting the first and second analysis items to axes, and the first or second analysis items are disposed adjacent to each other based on their correlations between each other.

The results are in forms of at least one of accuracy, confidence, probability and truth value.

An apparatus includes a generator configured to generate results of analysis items based on measured data. The apparatus further includes a controller configured to determine the analysis items as variants such that correlated ones of the analysis items are adjacent to each other, and display the results and the analysis items.

The analysis items include at least one of kinds of possible diseases, pathological causes of the possible diseases, and positions of the possible diseases.

Other features and aspects will be apparent from the following detailed description, the drawings, and the claims.

### BRIEF DESCRIPTION OF THE DRAWINGS

FIGS. 1A to 1C are diagrams illustrating examples of displaying analysis results of medical measured data using radial graphs, respectively.

FIG. 2 is a diagram illustrating an example of three-dimensionally displaying an analysis result of medical measured data.

FIG. 3 is a diagram illustrating an example of displaying an analysis result of medical measured data that is accumulated as time elapses.

FIG. 4 is a diagram illustrating another example of displaying an analysis result of medical measured data using a bar graph.

FIG. 5 is a flowchart illustrating an example of a method of displaying an analysis result of medical measured data.

FIG. 6 is a block diagram illustrating an example of an apparatus configured to display an analysis result of medical measured data.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. Also, descriptions of well-known functions and constructions may be omitted for increased clarity and conciseness.

Various measured medical data may be required to diagnose diseases. The measured medical data may include, for example, an electrocardiogram, a blood pressure, a body temperature, an oxygen saturation, MRI data and CT data, which may be obtained by directly measuring a patient's biological signals. The measured medical data may further include, for example, blood glucose, blood test data and diabetic data, which may be obtained by collecting substances from the patient's body and performing biochemical tests on the collected body substances. The measured medical data may be analyzed, thereby obtaining one or more results of one or more analysis items.

These analysis items may include, for example, kinds of diseases, causes of the diseases, positions of the diseases, and the like. Result values of the analysis items may be derived in the forms of accuracy, confidence, probability and/or truth value (e.g., percentages) based on analysis algorithms. Various classifiers may be used to classify the measured medical data into respective analysis items based on the analysis algorithms. For example, the classifiers may be configured with combinations of a fuzzy set classifier, a support vector machine (SVM) and a binary classifier including a decision tree, etc. To diagnose diseases, it may be important to not simply list the result values of the analysis items, but to visually display the result values so that medical staffs may use the result values more intuitively.

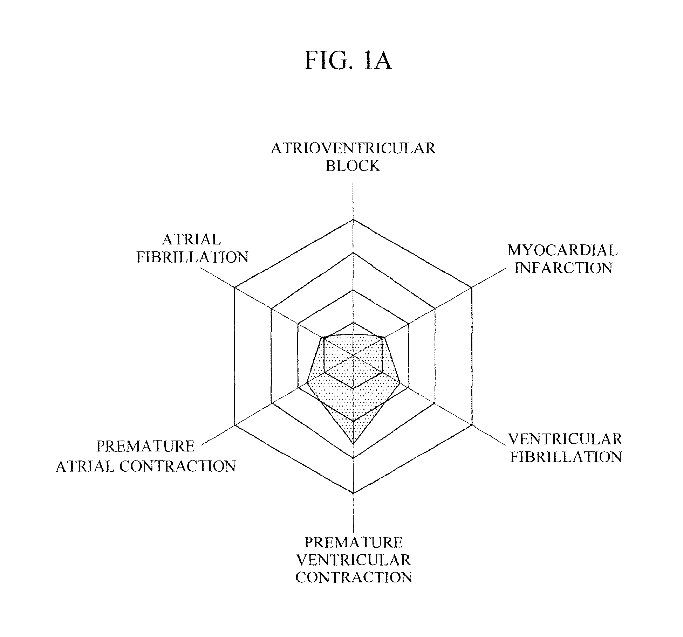

FIGS. 1A to 1C are diagrams illustrating examples of displaying analysis results of medical measured data using radial graphs, respectively. For example, ECG measured data may be analyzed, thereby obtaining analysis result values on kinds of cardiac disorders, pathological causes of the cardiac disorders and positions of the cardiac disorders. As the example illustrated in FIG. 1A shows, a radial graph may be displayed that includes kinds of diseases (e.g., the cardiac disorders) respectively as axes. The kinds of the possible diseases may include, for example, an atrioventricular block, a myocardial infarction, a ventricular fibrillation, a premature ventricular contraction, a premature atrial contraction and an atrial fibrillation. An analysis result value (e.g., a percentage) of each of these analysis items may be displayed as a distance value from the center of the radial graph.

Each of the axes (e.g., the kinds of the diseases) in the radial graph may be disposed so that correlated diseases are adjacent to each other. For example, the atrioventricular block may be adjacent to the myocardial infraction if they are correlated, e.g., similar to each other. Since the correlated diseases may be disposed on the adjacent axes, respectively, causes of the diseases may be more visually identified. For example, in the radial graph, the result values of the analysis items, occupy broad areas in directions in which the premature atrial contraction, the premature ventricular contraction and the ventricular fibrillation are positioned, respectively. As a result, based on the radial graph, medical staffs may diagnose that the possibility of the premature ventricular contraction is high.

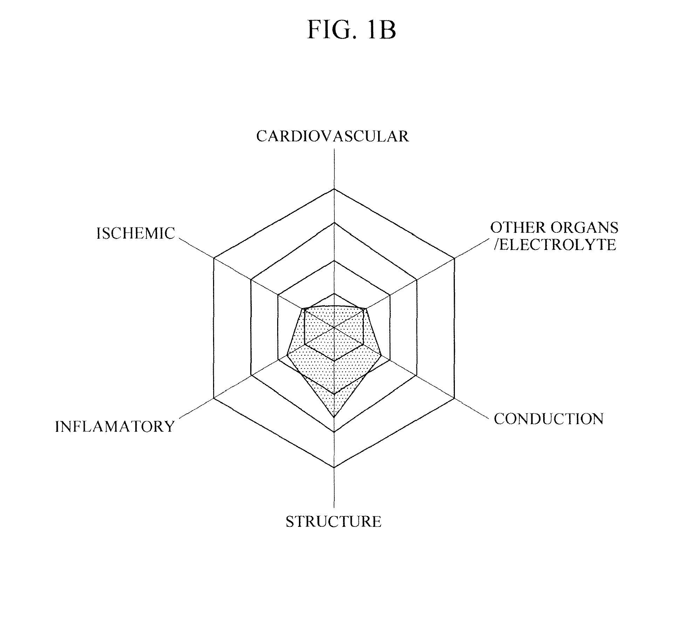

As illustrated in FIG. 1B, a radial graph may be displayed that includes analysis items of pathological causes of the diseases as axes, and these analysis items may be disposed based on correlations of the pathological causes of the diseases. For example, the pathological causes of the diseases (e.g., cardiac disorders) may be classified into cardiovascular, other organs/electrolyte, conduction, structure, inflammatory and ischemic. An analysis result value (e.g., a percentage) of each of these analysis items may be displayed as a distance value from the center of the radial graph.

Each of the pathological causes of the diseases in the radial graph may be disposed so that correlated causes of the diseases are adjacent to each other. For example, cardiovascular may be adjacent to other organs/electrolyte if they are correlated, e.g., similar to each other. As a result, based on the radial graph, medical staffs may diagnose that the possibility of a problem in the structure of a heart is higher than in other cases.

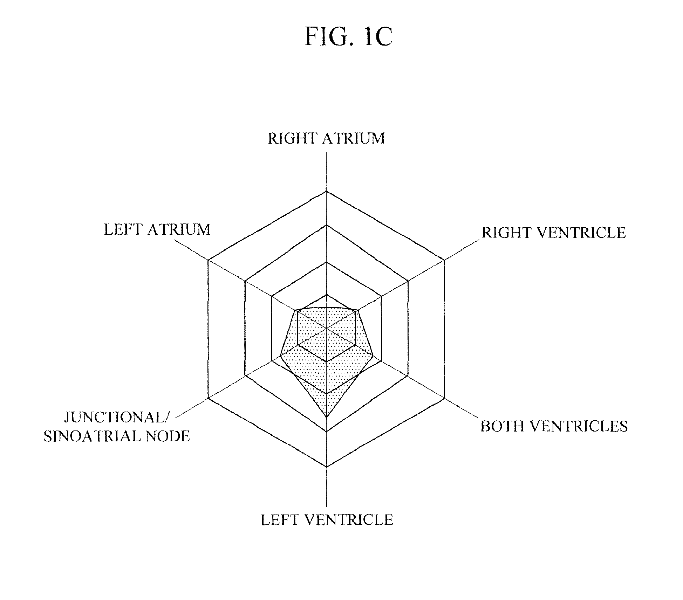

Referring now to FIG. 1C, a radial graph may be displayed that includes positions of the diseases as axes. For example, the positions of the diseases with respect to a heart may include a right atrium, a right ventricle, both ventricles, a left ventricle, a junctional/sinoatrial node and a left atrium. Their analysis result values (e.g., percentages) may be displayed as a distance value from the center of the radial graph.

Each of the positions of the diseases in the radial graph may be disposed so that proximate positions of the diseases are adjacent to each other. For example, the right atrium may be adjacent to the right ventricle if they are, e.g., in close proximity to each other. Based on the radial chart, medical staffs may diagnose that the possibility of a problem in the left ventricle is higher than in other cases.

In the examples of FIGS. 1A to 1C described above, each of the axes may be an analysis item, and the axes may be disposed in the order obtained by defining correlations between the analysis items. That is, an undirected radial graph may be obtained by defining a name (i.e., an analysis item) of each of axes as a vertex and setting a correlation between the axes to a value of an edge between nodes. In this example of the undirected radial graph, "nodes" refer to values of each analysis item, and "edges" refer to lines that connect the nodes. The order of disposing the axes may be determined based on Hamiltonian touring sequences that evaluate a touring path having a greatest sum of values of edges on the path when touring a vertex.

In the example of FIG. 1A, the correlation between the diseases may be defined as, for example, a coincidence probability of the diseases. The coincidence probability of the diseases may be defined based on a Jaccard index of two diseases from diseased patient data in a target population that is secured as statistical data. The Jaccard index may be defined as a value obtained by dividing a number of patients having both diseases by a number of elements in a union of patients having a single disease.

The pathological causes of the diseases may be displayed as a pathological tree structure including hierarchical structures based on several conventional methods. A branch of the tree structure may define a level based on a distance from each root node, and may define a weight value in inverse proportion to the level. The correlation between the pathological causes of the diseases may be defined as a value in proportion to a reciprocal of a distance between trees (i.e., a sum of weight values of the trees) in the pathological tree structure.

The correlation between the positions of the diseases may be set based on physical proximities. For example, the structure of human internal organs and blood vessels may be displayed as an undirected radial graph based on anatomic correlations. Weight values of edges may be set identical to one another or may be set to arbitrary values. The correlation between the positions of the diseases may be defined as a value in inverse proportion to a reciprocal of a distance on the radial graph.

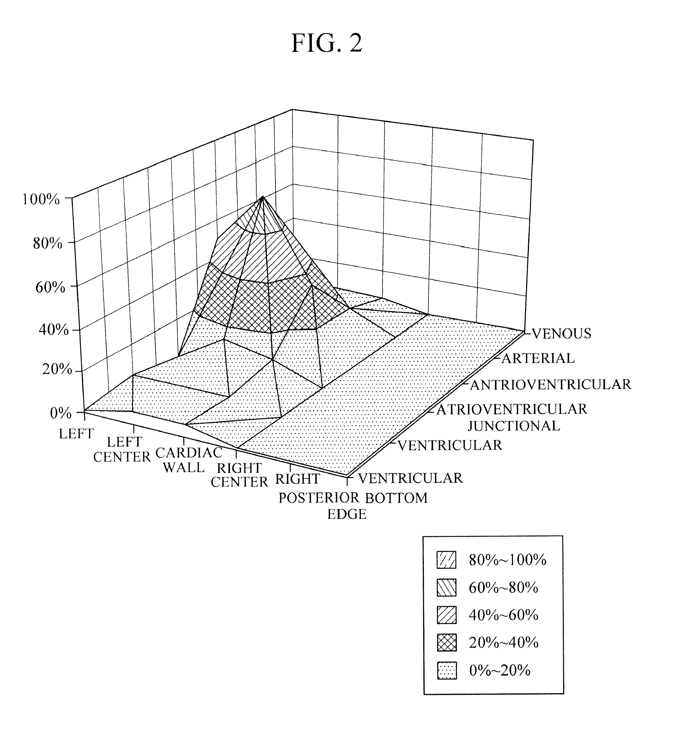

FIG. 2 is a diagram illustrating an example of three-dimensionally displaying an analysis result of medical measured data. That is, a three-dimensional graph may be displayed in place of the two-dimensional graphs illustrated in FIGS. 1A to 1C. In other words, measured medical data may be analyzed and subdivided into results (e.g., percentages of diseases) of first analysis items and second analysis items. The first and second analysis items may be set to respective X and Y axes, and the results of the first and second analysis items may be set to a Z-axis, thereby displaying the analyzed results as a three-dimensional graph. The first or second analysis items may be disposed adjacent to each other.

For example, the first analysis items may include relative positions from a heart, such as arterial, venous, atrioventricular, atrioventricular junctional, ventricular, and ventricular bottom. The second analysis items may include relative positions from one object, for example, left, left center, cardiac wall, right center, right and posterior edge. As such, a more exact position of the disease may be three-dimensionally diagnosed through the graph in which the position of the disease may be displayed, e.g., as a position in cardiac blood vessels.

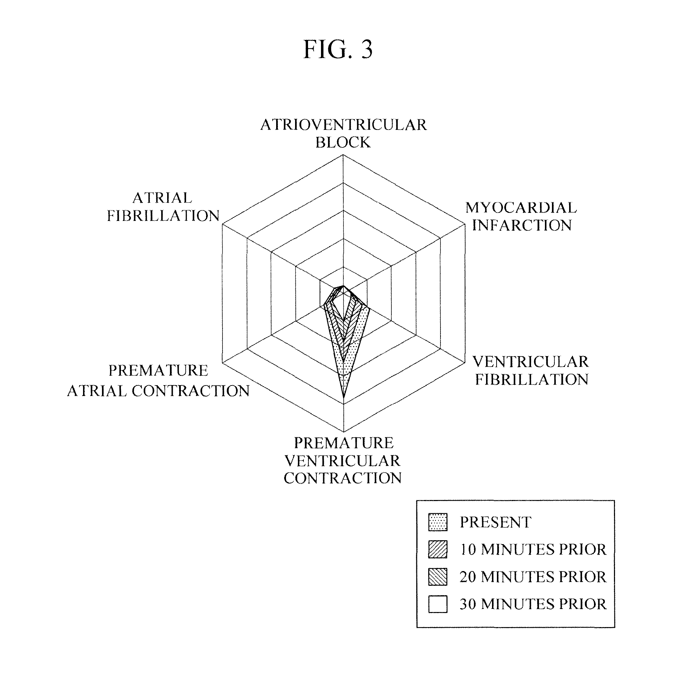

FIG. 3 is a diagram illustrating an example of displaying an analysis result of medical measured data that is accumulated as time elapses. The analysis result of the measured medical data may be changed as time elapses. The analysis result may be displayed by accumulating analysis results over a certain period of time. For example, the analysis result may be displayed by accumulating analysis results from the present and 10 minutes, 20 minutes and 30 minutes prior. Accordingly, the analysis result obtained by accumulating analysis results may obtain a higher reliability than that obtained by analyzing only data at a certain instance. As discussed above with respect to FIGS. 1A to 1C, analysis items of the axes (e.g., an atrioventricular block and a myocardial infraction) may be disposed adjacent to each other based on their correlations between each other.

Referring back to FIG. 3, the analysis result obtained by analyzing all measured medical data inputted up to the present may be synthetically displayed, or a time setting value may be inputted by a user through a separate input interface so as to display an analysis result during a corresponding period of time. The analysis result for each period may be obtained by dividing a measured value for each predetermined period, and the analysis result may be displayed by accumulating corresponding results on one graph. In addition, the analysis result may be displayed using various methods including a method of displaying result values for analysis items in several colors as time elapses, a method of displaying result values for analysis items as several brightnesses, patterns or line thicknesses, etc.

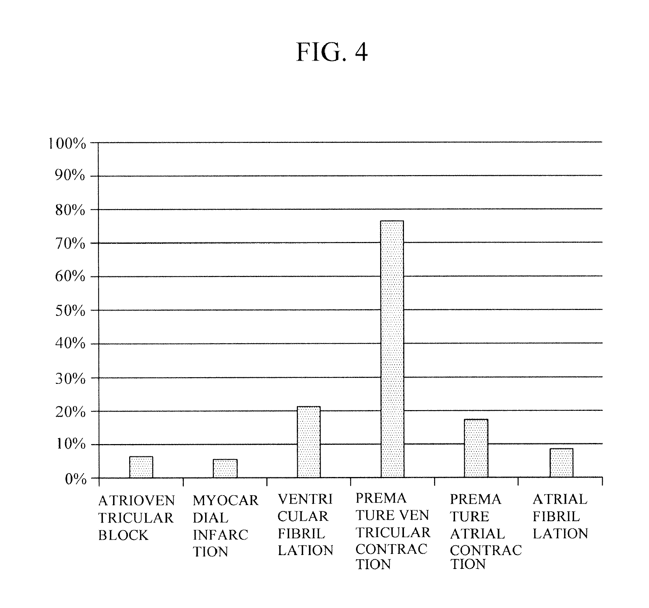

FIG. 4 is a diagram illustrating another example of displaying an analysis result of medical measured data using a bar graph. The analysis result may be displayed in the form of the bar graph as well as the radial graph (e.g., in FIG. 1A). Analysis items (e.g., an atrioventricular block and a myocardial infraction) having high correlations may be disposed adjacent to each other on an X-axis. Result values (e.g., percentages of diseases) of the analysis items may be set to a Y-axis. Since the analysis items may be disposed based on correlations between the analysis items, the bar graph may obtain a similar display result as the radial graph.

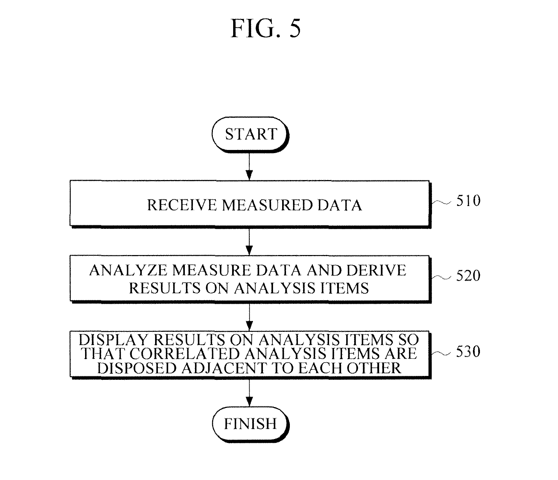

FIG. 5 is a flowchart illustrating an example of a method of displaying an analysis result of medical measured data. At step 510, measured medical data necessary for disease diagnosis, including, e.g., analysis data of a patient's biological signals or body substances, may be received. For example, the measured medical data may include data obtained by directly measuring the patient's biological signals and data obtained by collecting substances from the patient's body and performing biochemical tests on the collected body substances.

At step 520, the received measured medical data may be analyzed, and one or more results of one or more analysis items may be derived based on the analyzed measured medical data. For example, the analysis items may include a name of a disease, a cause of the disease and a position of the disease. The result values of the analysis items may be derived in the forms of accuracy, confidence, probability and/or truth value (e.g., percentages) based on analysis algorithms.

At step 530, the results of the analysis items may be displayed by setting each of the analysis items as a variant in a graph and allowing correlated analysis items to be disposed adjacent to each other in the graph. For example, the results of the analysis items may be displayed in a radial graph in which variants (i.e., the analysis items) may be axes. The analysis items may be disposed based on correlations (e.g., similarities and/or physical proximities) between the analysis items, corresponding to the respective axes.

In examples, the analysis items may include kinds of possible diseases, and may be disposed based on correlations between the diseases. Alternatively, the analysis items may include pathological causes of the possible diseases, and may be disposed based on correlations of the pathological causes of the diseases. Alternatively, the analysis items may include positions of the possible diseases, and may be disposed based on physical proximities at the positions of the diseases. The display results according to these examples may be the same as described with reference to FIGS. 1A to 1C.

The results of the analysis items may be accumulated as time elapses, and the accumulated results may be displayed. The display results according to this example may be the same as described with reference to FIG. 3. In another example, the display results may be displayed three-dimensionally. As described with reference to FIG. 2, measured medical data may be analyzed and subdivided into results of first analysis items and second analysis items. The first and second analysis items may be set to respective X and Y axes, thereby displaying the analyzed results as a three-dimensional graph. In this case, the first or second analysis items may be disposed adjacent to each other according to their correlations between each other.

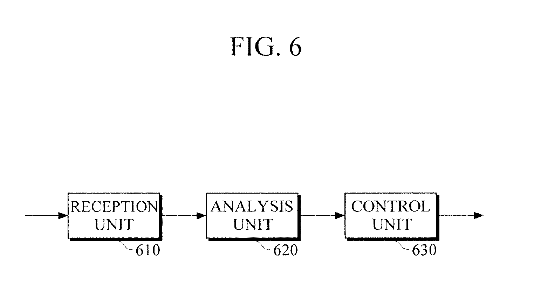

FIG. 6 is a block diagram illustrating an example of an apparatus configured to display an analysis result of medical measured data. The apparatus may include a reception unit 610, an analysis unit 620 and a control unit 630. The reception unit 610 may receive measured medical data necessary for disease diagnosis, including, e.g., analysis data of a patient's biological signals or body substances. The measured medical data may include, for example, data obtained by directly measuring the patient's biological signals and data obtained by collecting substances from the patient's body and performing biochemical tests on the collected body substances.

The analysis unit 620 (e.g., a results generator) may analyze received measured medical data, and may derive one or more results of one or more analysis items based on the analyzed measured medical data. For example, the analysis items may include a name of a disease, a cause of the disease and a position of the disease. The result values of the analysis items may be derived in the forms of accuracy, confidence, probability and/or truth value (e.g., percentages) based on analysis algorithms.

The control unit 630 (e.g., a results controller) may display the results of the analysis items by setting each of the analysis items as a variant in a graph and allowing correlated analysis items to be disposed adjacent to each other in the graph. For example, the results of the analysis items may be displayed as a radial graph in which variants (e.g., the analysis items) may be axes. The analysis items may be disposed based on correlations (e.g., similarities and/or physical proximities) between the analysis items, corresponding to the respective axes.

In examples, the analysis items may include kinds of possible diseases, and may be disposed based on correlations between the diseases. Alternatively, the analysis items may include pathological causes of the possible diseases, and may be disposed based on correlations of the pathological causes of the diseases. Alternatively, the analysis items may include positions of the possible diseases, and may be disposed based on physical proximities at the positions of the diseases. The display results according to these examples may be the same as described with reference to FIGS. 1A to 1C.

The results of the analysis items may be accumulated as time elapses, and the accumulated results may be displayed. The display result according to this example may be the same as described with reference to FIG. 3. In another example, the display results may be displayed three-dimensionally. As described with reference to FIG. 2, measured medical data may be analyzed and subdivided into results of first analysis items and second analysis items, and the first and second analysis items may be set to respective X and Y axes, thereby displaying the analyzed results as a three-dimensional graph. In this case, the first or second analysis items may be disposed adjacent to each other according to their correlations between each other.

According to the teachings above, there is provided a method and apparatus in which kinds of possible diseases, causes of the diseases and positions of the diseases may be visually displayed so that these analysis items are correlated with one another. The display results may be provided to medical staffs, thereby helping the medical staffs with the diagnosis and prescription of a disease.

The units described herein may be implemented using hardware components and software components. For example, microphones, amplifiers, band-pass filters, audio to digital convertors, and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors. As used herein, a processing device configured to implement a function A includes a processor programmed to run specific software. In addition, a processing device configured to implement a function A, a function B, and a function C may include configurations, such as, for example, a processor configured to implement both functions A, B, and C, a first processor configured to implement function A, and a second processor configured to implement functions B and C, a first processor to implement function A, a second processor configured to implement function B, and a third processor configured to implement function C, a first processor configured to implement function A, and a second processor configured to implement functions B and C, a first processor configured to implement functions A, B, C, and a second processor configured to implement functions A, B, and C, and so on.

The software may include a computer program, a piece of code, an instruction, or some combination thereof, for independently or collectively instructing or configuring the processing device to operate as desired. Software and data may be embodied permanently or temporarily in any type of machine, component, physical or virtual equipment, non-transitory computer storage medium or device, or in a propagated signal wave capable of providing instructions or data to or being interpreted by the processing device. The software also may be distributed over network coupled computer systems so that the software is stored and executed in a distributed fashion. In particular, the software and data may be stored by one or more non-transitory computer readable recording mediums. The computer readable recording medium may include any data storage device that can store data which can be thereafter read by a computer system or processing device. Examples of the computer readable recording medium include read-only memory (ROM), random-access memory (RAM), CD-ROMs, magnetic tapes, floppy disks, optical data storage devices. Also, functional programs, codes, and code segments for accomplishing the example embodiments disclosed herein can be easily construed by programmers skilled in the art to which the embodiments pertain based on and using the flow diagrams and block diagrams of the figures and their corresponding descriptions as provided herein.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

The disclosure provides the following further aspects listed below and counted from one. The aspect of a method of displaying an analysis result of measured data, the method comprising: analyzing measured data received for disease diagnosis and deriving results of analysis items based on the analyzed measured data; and displaying the results by setting each of the analysis items as a variant and allowing correlated ones of the analysis items to be disposed adjacent to each other.
The method according to the first aspect, wherein the displaying of the results comprises displaying the results in a radial graph in which variants comprise axes, and the analysis items are disposed based on correlations between the analysis items, corresponding to the respective axes. The method according to the first aspect, wherein the analysis items comprise kinds of possible diseases, and are disposed based on correlations between the diseases. The method according to the first aspect, wherein the analysis items comprise pathological causes of possible diseases, and are disposed based on correlations between the pathological causes of the diseases. The method according to the first aspect, wherein the analysis items comprise positions of possible diseases, and are disposed based on physical proximities at the positions of the diseases. The method according to the first aspect, wherein the displaying of the results comprises displaying the results accumulated as time elapses. The method according to the first aspect, wherein the deriving of the results comprises subdividing the analyzed measured data into results of first analysis items and second analysis items. The method according to the previous aspect wherein the displaying of the results comprises three-dimensionally displaying the results by respectively setting the first and second analysis items to X and Y axes, and the first or second analysis items are disposed adjacent to each other based on their correlations between each other. The method according to the first aspect, wherein the results are in forms of at least one of accuracy, confidence, probability and truth value.
The aspect of an apparatus configured to display an analysis result of measured data, the apparatus comprising: an analysis unit configured to analyze measured data received for disease diagnosis, and derive results of analysis items based on the analyzed measured data; and a control unit configured to display the results by setting each of the analysis items as a variant and allowing correlated ones of the analysis items to be disposed adjacent to each other.
The apparatus according to the tenth aspect, wherein the control unit displays the results in a radial graph in which variants comprise axes, and the analysis items are disposed based on correlations between the analysis items, corresponding to the respective axes. The apparatus according to the tenth aspect, wherein the analysis items comprise kinds of possible diseases, and are disposed based on correlations between the diseases. The apparatus according to the tenth aspect, wherein the analysis items comprise pathological causes of possible diseases, and are disposed based on correlations between the pathological causes of the diseases. The apparatus according to the tenth aspect, wherein the analysis items comprise positions of possible diseases, and are disposed based on physical proximities at the positions of the diseases. The apparatus according to the tenth aspect, wherein the control unit displays the results accumulated as time elapses. The apparatus according to the tenth aspect, wherein the control unit subdivides the analyzed measured data into results of first analysis items and second analysis items. The apparatus according to the previous aspect wherein the control unit three-dimensionally displays the results by respectively setting the first and second analysis items to axes, and the first or second analysis items are disposed adjacent to each other based on their correlations between each other. The apparatus according to the tenth aspect, wherein the results are in forms of at least one of accuracy, confidence, probability and truth value.
The aspect of an apparatus comprising:a generator configured to generate results of analysis items based on measured data; and a controller configured to determine the analysis items as variants such that correlated ones of the analysis items are adjacent to each other, and display the results and the analysis items. The apparatus according to the previous aspect, wherein the analysis items comprise at least one of kinds of possible diseases, pathological causes of the possible diseases, and positions of the possible diseases.

## Claims

1. A method of displaying an analysis result of measured data, the method comprising:
analyzing measured data received for disease diagnosis and deriving results of analysis items based on the analyzed measured data; and
displaying the results by setting each of the analysis items as a variant and allowing correlated ones of the analysis items to be disposed adjacent to each other.

2. The method of claim 1, wherein the displaying of the results comprises displaying the results in a radial graph in which variants comprise axes, and the analysis items are disposed based on correlations between the analysis items, corresponding to the respective axes.

3. The method of claim 1, wherein the analysis items comprise kinds of possible diseases, and are disposed based on correlations between the diseases.

4. The method of claim 1, wherein the analysis items comprise pathological causes of possible diseases, and are disposed based on correlations between the pathological causes of the diseases.

5. The method of claim 1, wherein the analysis items comprise positions of possible diseases, and are disposed based on physical proximities at the positions of the diseases.

6. The method of claim 1, wherein the displaying of the results comprises displaying the results accumulated as time elapses.

7. The method of claim 1, wherein the deriving of the results comprises subdividing the analyzed measured data into results of first analysis items and second analysis itemswherein the displaying of the results comprises three-dimensionally displaying the results by respectively setting the first and second analysis items to X and Y axes, and the first or second analysis items are disposed adjacent to each other based on their correlations between each other.

8. The method of claim 1, wherein the results are in forms of at least one of accuracy, confidence, probability and truth value.

9. An apparatus configured to display an analysis result of measured data, the apparatus comprising:
an analysis unit configured to analyze measured data received for disease diagnosis, and derive results of analysis items based on the analyzed measured data; and
a control unit configured to display the results by setting each of the analysis items as a variant and allowing correlated ones of the analysis items to be disposed adjacent to each other.

10. The apparatus of claim 9, wherein the control unit displays the results in a radial graph in which variants comprise axes, and the analysis items are disposed based on correlations between the analysis items, corresponding to the respective axes.

11. The apparatus of claim 9, wherein the analysis items comprise kinds of possible diseases, and are disposed based on correlations between the diseases.

12. The apparatus of claim 9, wherein the analysis items comprise pathological causes of possible diseases, and are disposed based on correlations between the pathological causes of the diseases.

13. The apparatus of claim 9, wherein the analysis items comprise positions of possible diseases, and are disposed based on physical proximities at the positions of the diseases.

14. The apparatus of claim 9, wherein the control unit displays the results accumulated as time elapses.

15. The apparatus of claim 9, wherein the control unit subdivides the analyzed measured data into results of first analysis items and second analysis items; and wherein the control unit three-dimensionally displays the results by respectively setting the first and second analysis items to axes, and the first or second analysis items are disposed adjacent to each other based on their correlations between each other.

16. The apparatus of claim 9, wherein the results are in forms of at least one of accuracy, confidence, probability and truth value.
